# EUROPEAN PATENT SPECIFICATION

(11) **EP 4 387 525 B1**
(45) Date of publication and mention of the grant of the patent: **29.04.2026**
(21) Application number: 22761741.2
(22) Date of filing: 02.08.2022
(51) Int. Cl.: A61B 5/20, A61F 5/44, G01F 1/00, A61B 5/00

(54) **URINARY OUTPUT MONITORING SYSTEM**
URINAUSGABEÜBERWACHUNGSSYSTEM
SYSTÈME DE SURVEILLANCE DE SORTIE URINAIRE

(30) Priority: 23.08.2021 US 202163236062 P
(43) Date of publication of application: 26.06.2024
(60) Divisional application: 26162487.8
(73) Proprietor: C. R. Bard, Inc., Franklin Lakes, NJ 07417 (US)
(72) Inventor: COMPTON, Clayton, L., Covington, GA 30016 (US); TARR, Matthew, R., Ponte Vedra, FL 32081 (US); MEYER, Andrew, L., Atlanta, GA 30316 (US)
(74) Representative: Hoffmann Eitle
(86) International application number: PCT/US2022/039191
(87) International publication number: WO 2023/027871

(56) References cited:
- WO-A1-2021/119307
- US-A1- 2008 058 630
- US-A1- 2017 113 000
- US-A1- 2020 064 172

## Description

### PRIORITY

This application claims the benefit of priority to U.S. Provisional Application No. 63/236,062, filed August 23, 2021.

### BACKGROUND

Urinary output monitoring systems can be used to monitor the urine output volume and urine flow rate of a patient. Most urinary output monitoring systems are bulky, requiring precious floor space. Furthermore, most urinary output monitoring systems are not mobile, meaning the systems are unable to monitor urinary output while a patient is being transported through a hospital environment. It would be beneficial to the clinician and the patient to have a urinary output monitoring system that is mobile and able to capture a patient's entire urine output including during transport. Disclosed herein is an automated urinary output monitoring system and method of use that addresses the foregoing. US 2017/113000 A1 discloses a urinary catheter including a sensing unit for sensing flow of urine along the catheter. US 2020/0064172 A1 discloses a system for collecting urine that includes a flow sensor.

### SUMMARY

Disclosed herein is a urinary output device, including a urinary catheter coupled with a urine collection bag via a drainage tube extending between the urinary catheter and the urine collection bag and a flow meter in line with the drainage tube. The flow meter is configured to determine a flow rate of urine flowing from the urinary catheter to the urine collection bag, and the flow meter includes a flow meter console that includes one or more processors and a non-transitory computer-readable medium having stored thereon flow meter logic that, when executed by the one or more processors, causes operations of the flow meter. The operations include determining the flow rate of urine and wirelessly transmitting to a monitor (i) urine flow rate data and (ii) a flow meter identifier stored in the non-transitory computer-readable medium.

In some embodiments of the device, the catheter, the drainage tube, and the urine collection bag define a pre-connected closed fluid system. In some embodiments, the urinary output device further includes a sample port coupled the drainage tube, where the sample is configured to enable a clinician to draw a sample of the urine from the drainage tube via a volumetric device.

In some embodiments of the device, the flow meter identifier includes alphanumeric characters, special characters, spaces, or combinations thereof.

In some embodiments of the device, the flow meter console includes a battery.

In some embodiments of the device, wherein urinary catheter, the drainage tube, and the urine collection bag define a pre-connected closed fluid system.

In some embodiments of the device, the flow meter is disposable.

These and other features of the concepts provided herein will become more apparent to those of skill in the art in view of the accompanying drawings and following description, which describe particular embodiments of such concepts in greater detail.

### DRAWINGS

A more particular description of the present disclosure will be rendered by reference to specific embodiments thereof that are illustrated in the appended drawings. It is appreciated that these drawings depict only typical embodiments of the invention and are therefore not to be considered limiting of its scope. Example embodiments of the invention will be described and explained with additional specificity and detail through the use of the accompanying drawings in which:
FIG. 1 illustrates a perspective view of a urinary output monitoring system, in accordance with some embodiments.
FIG. 2 illustrates a block diagram of some components of the urinary output monitoring system of FIG. 1 including a monitor and a monitor console, in accordance with some embodiments.
FIG. 3 illustrates a block diagram of some components of a flow meter of the urinary output monitoring system of FIG. 1 including a flow meter console, in accordance with some embodiments.
FIG. 4 illustrates a perspective view of the monitor of the urinary output monitoring system of FIG. 1, in accordance with some embodiments.
FIGS. 5A-5D are illustrations of the urinary output monitoring system of FIG. 1 at various stages of an exemplary method of monitoring urinary output of a patient, in accordance with some embodiments.
FIG. 6 illustrates a flow chart of the exemplary method of monitoring urinary output of a patient, in accordance with some embodiments.

### DESCRIPTION

Before some particular embodiments are disclosed in greater detail, it should be understood that the particular embodiments disclosed herein do not limit the scope of the concepts provided herein. It should also be understood that a particular embodiment disclosed herein can have features that can be readily separated from the particular embodiment and optionally combined with or substituted for features of any of a number of other embodiments disclosed herein.

Regarding terms used herein, it should also be understood the terms are for the purpose of describing some particular embodiments, and the terms do not limit the scope of the concepts provided herein. Ordinal numbers (e.g., first, second, third, etc.) are generally used to distinguish or identify different features or steps in a group of features or steps, and do not supply a serial or numerical limitation. For example, "first," "second," and "third" features or steps need not necessarily appear in that order, and the particular embodiments including such features or steps need not necessarily be limited to the three features or steps. Labels such as "left," "right," "top," "bottom," "front," "back," and the like are used for convenience and are not intended to imply, for example, any particular fixed location, orientation, or direction. Instead, such labels are used to reflect, for example, relative location, orientation, or directions. Singular forms of "a," "an," and "the" include plural references unless the context clearly dictates otherwise.

Unless defined otherwise, all technical and scientific terms used herein have the same meaning as commonly understood by those of ordinary skill in the art.

Any methods disclosed herein comprise one or more steps or actions for performing the described method. The method steps and/or actions may be interchanged with one another. In other words, unless a specific order of steps or actions is required for proper operation of the embodiment, the order and/or use of specific steps and/or actions may be modified.

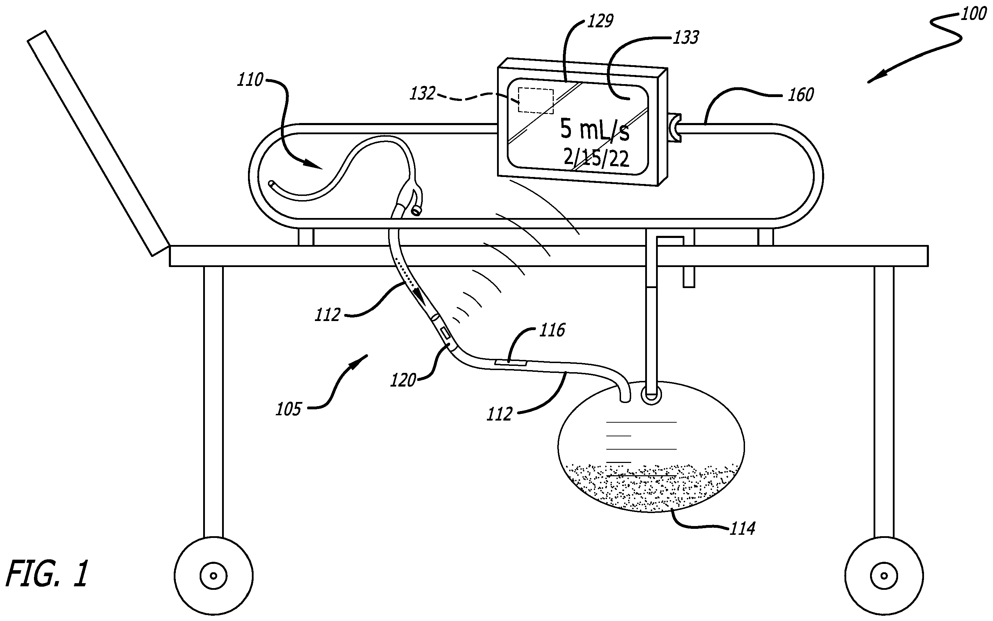

FIG. 1 illustrates a perspective view of a urinary output monitoring system 100, in accordance with some embodiments. In some embodiments, the urinary output monitoring system ("system") may include generally include a urinary output collection device (device) 105 communicatively coupled with a monitor 129. The urinary output collection device 105 includes a urinary catheter 110 in fluid communication with a drainage tube 112. In some embodiments, the drainage tube 112 may be in fluid communication with a fluid (urine) collection bag 114. The monitor 129 includes a display 133 and a monitor console 132, and the monitor 129 may be coupled to a securing surface 160. In some embodiments, the securing surface 160 may include a portion of a hospital bed. The system 100 (or more specifically the device 105) may further include a flow meter 120 having a flow meter console 122 in wireless communication with the monitor console 132. Exemplary wireless communication modalities can include WiFi, Bluetooth, Near Field Communications (NFC), cellular Global System for Mobile Communication ("GSM"), electromagnetic (EM), radio frequency (RF), combinations thereof, or the like. In some embodiments, the flow meter 120 may be located within the drainage tube 112, integrated into the drainage tube 112 or may be configured to be placed in line between the urinary catheter 110 and the fluid collection bag 114. The monitor 129 may be configured to display determined flow rate values, as will be described in more detail herein.

In some embodiments, the device 105 may define a closed pre-connected device 105 (e.g., the urinary catheter 110 may be preconnected to the fluid collection bag 114 by the drainage tube 112 having the in line flow meter 120). The urinary catheter 110 may be inserted into a patient, while the drainage tube 112 directs urine from the urinary catheter 110 to the fluid collection bag 114. The patient may void a volume of fluid (e.g., urine) that travels through the drainage tube 112 to the fluid collection bag 114 by passive gravity flow, negative pressure flow or the like. As the volume of fluid travels to the fluid collection bag 114, the volume of fluid may pass through the flow meter 120. The flow meter 120 may be configured to determine the one or more flow rate values of the volume of fluid before the volume of fluid reaches the fluid collection bag 114 (i.e., generate urine flow rate data). The flow meter 120 may transmit the one or more flow rate values to the monitor console 132. Each time the patient voids a volume of fluid, the flow meter 120 may be configured to determine the one or more flow rate values and transmit the one or more flow rate values to the monitor console 132.

Additionally, the flow meter 120 may include a flow meter identifier that may be transmitted to the monitor console 132. The flow meter identifier may be used by the monitor console 132 to correlate received flow rate values with the flow meter identifier similar to a patient identifier. In some embodiments, the flow meter identifier may include alphanumeric characters, special characters (e.g., punctuation marks, Unicode characters, pictographs, or the like), spaces, and combinations thereof within the flow meter identifier. The flow meter identifier may include a serial number of the flow meter 120. In some embodiments, the flow meter identifier may also be a product key for the monitor console 132, in that the monitor console 132 may not receive the determined flow rate values from the flow meter 120 until the correct flow meter identifier is received by the monitor console 132. Advantageously, the flow meter identifier link the urine flow rate data to the flow mater 120 and allows urine flow rate data to be aggregated with previous urine flow rate data that will be described in more detail herein.

In some embodiments, the device 105, may include a sample port 116 coupled the drainage tube, where the sample port 116 is configured to enable a clinician to draw a sample of the urine from the drainage tube via a volumetric device, such as a syringe, for example.

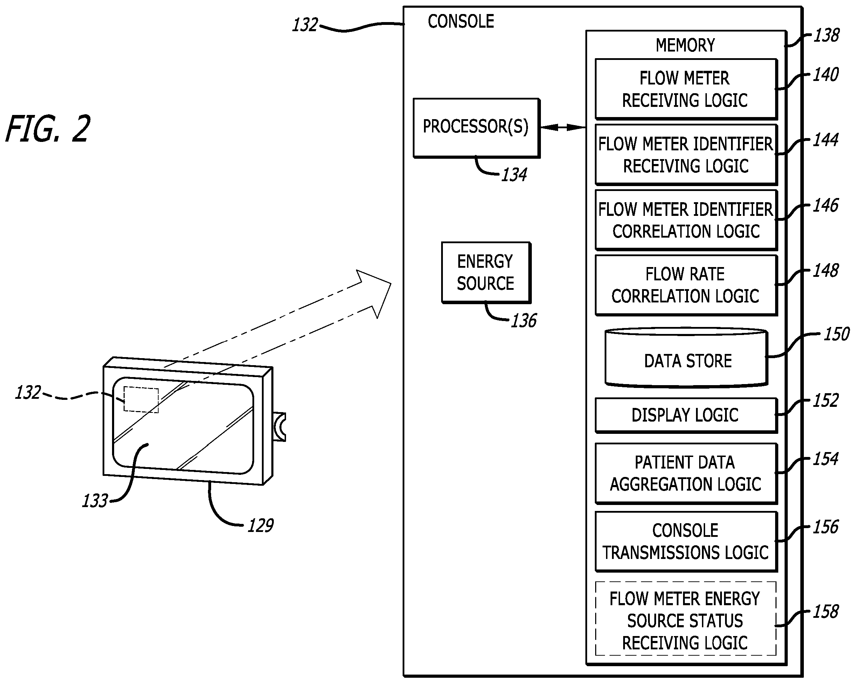

FIG 2 illustrates a block diagram of some components of the system 100 including the monitor console 132 and the monitor 129, in accordance with some embodiments. In some embodiments, the monitor console 132 may be coupled to the monitor 129 or integrated into the monitor 129. In some embodiments, the monitor console 132 includes one or more processors 134, a first energy source 136, non-transitory computer readable medium ("memory") 138 and a plurality of logic modules. In some embodiments, the first energy source 136 may be an external energy source (e.g., facility power) wired to the system 100 (e.g., a power cord from the monitor 129 to a standard power outlet or the like). In some embodiments, the first energy source 136 may include a rechargeable battery (e.g., a backup battery) that may be configured to be recharged when the monitor 129 is plugged into the power outlet. In some embodiments, the plurality of logic modules may include a flow meter receiving logic 140, a flow meter identifier receiving logic 144, a flow meter identifier correlation logic 146, a flow rate correlation logic 148, a first data store 150, a display logic 152, a patient data aggregation logic 154, a console transmissions logic 156, and a flow meter energy source status receiving logic 158.

In some embodiments, the flow meter receiving logic 140 may be configured to wirelessly receive flow meter values (urine flow rate data) from the flow meter 120. In some embodiments, the flow meter receiving logic 140 may automatically determine and commence communication with the flow meter 120. In some embodiments, the flow meter receiving logic 140 may be configured receive the flow rate values from the flow meter 120. In some embodiments, the flow meter identifier receiving logic 144 may be configured to receive the flow meter identifier from the flow meter 120 . In some embodiments, the flow meter identifier correlation logic 146 may be configured to correlate the flow meter identifier with a patient identification (e.g., a medical records number or the like), the flow rate values or a combination thereof. In some embodiments, the flow rate correlation logic 148 may be configured to correlate the calculated flow rate value with a time of day value. The flow rate correlation logic 148 may also integrate the urine flow rate data with respect to time to calculate a volume of urine. In some embodiments, the flow rate correlation logic 148 may be configured to correlate the determined flow rate values with the time of day value in a {determined flow rate value, time of day value or correlated urine flow rate data}. In some embodiments, the first data store 150 may be configured to store each {determined flow rate value, time of day value} as well as the flow meter identifier. In some embodiments, the display logic 152 may be configured to transmit the determined flow rate value and time of day value to the monitor 129. In some embodiments, the patient data aggregation logic 154 may be configured to aggregate urine flow rate data (e.g., flow rate values and time of day values) with existing patient data or add urine flow rate data to a new patient identification. For example, if the system 100 is replaced, the new flow meter 120 may transmit new flow rate values to the monitor console 132 and the patient data aggregation logic 154 may be configured to add the new flow rate values to the existing patient data. In some embodiments, the console transmissions logic 156 may be configured to transmit all calculated urine flow rate data values and time of day values to an electronic medical records system. In some embodiments, the flow meter energy source status receiving logic 158 may be configured to receive the flow meter energy source status (battery level) from the flow meter 120 and may generate an alert if the flow meter energy source (battery level) is significantly decreasing in available power to power the flow meter 120, indicating the flow meter 120 needs to be replaced.

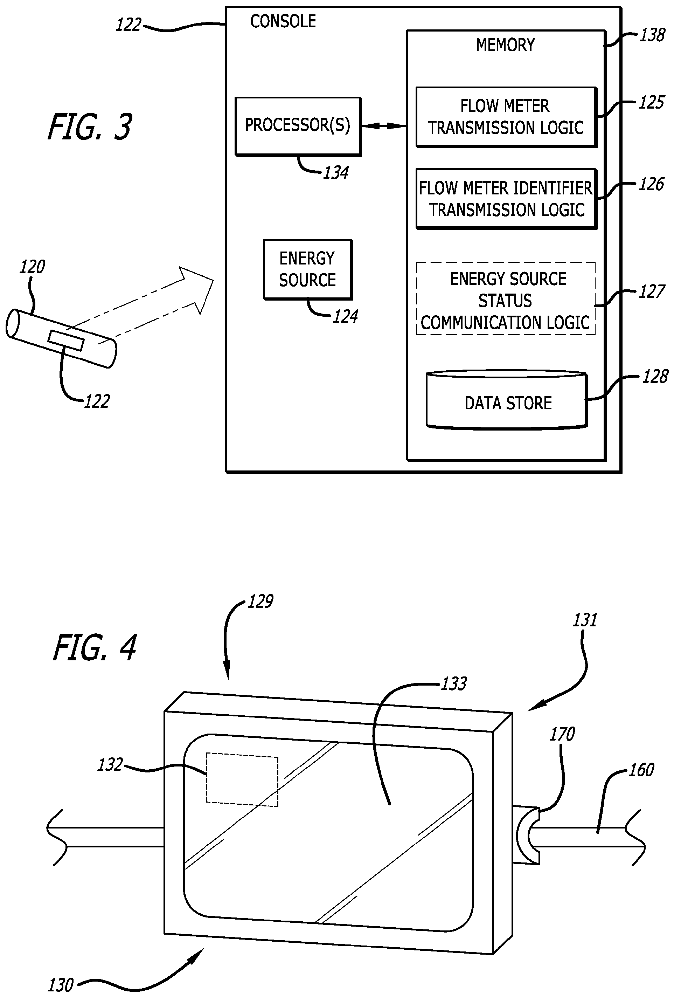

As illustrated in FIG. 3, in some embodiments, the flow meter 120 may include a standalone flow meter 120 that may be assembled in line with the drainage tube 112 or may be integrated into the drainage tube 112. In some embodiments, the flow meter 120 may be disposable. In some embodiments, the flow meter 120 may include the flow meter console 122 in wireless communication with the monitor console 132. The flow meter console 122 may include one or more processors 134, a second energy source 124, non-transitory computer readable medium ("memory") 138 and one or more logic modules. In some embodiments, the second energy source 124 may include a battery that is compatible with the lifetime use of the system 100. In some embodiments, the one or more logic modules may include a flow meter transmission logic 125, a flow meter identifier transmission logic 126, a second data store 128 and optionally, an energy source status communication logic 127. In some embodiments, the flow meter transmission logic 125 may be configured to wirelessly transmit flow rate values from the flow meter console 122 to the monitor console 132. In some embodiments, the flow meter transmission logic 125 may wireless transmit flow rate values as they are determined or may transmit flow rate values on an automatic or user defined timeline (e.g., once or more every hour). In some embodiments, the flow meter identifier transmission logic 126 may be configured to transmit the flow meter identifier to the monitor console 132. In some embodiments, the energy source status communication logic 127 may be configured to transmit to the monitor console 132 the status of the second energy source 124. In some embodiments, the energy source status communication logic 127 may be configured to transmit the status of the second energy source 124 as a percent of available battery left or in number of hours remaining for the battery. In some embodiments, the second data store 128 may be configured to store the flow rate values in situations where the flow meter 120 may lose wireless communication with the monitor console 132 or cannot transmit the flow rate values.

FIG. 4 illustrates a perspective view of the monitor 129, in accordance with some embodiments. In some embodiments, the monitor 129 may include a front side 130 and a back side 131, the back side 131 including a coupling device 170. The coupling device 170 may be configured to detachably couple the monitor 129 to the securing surface 160 (e.g., the hospital bed, an IV stand or the like). In some embodiments, the coupling device 170 may include one or more hinge clamps. The monitor 129 includes the display 133 which may include a touch screen. In some embodiments, the monitor 129 may include the monitor console 132 therein. In some embodiments, the coupling device 170 may be integrated into the monitor 129 or coupled to the monitor 129. The coupling device 170 may allow the monitor 129 to be suspended from the securing surface 160. In some embodiments, the coupling device 170 may prevent sag or movement of the monitor 129 from the securing surface 160.

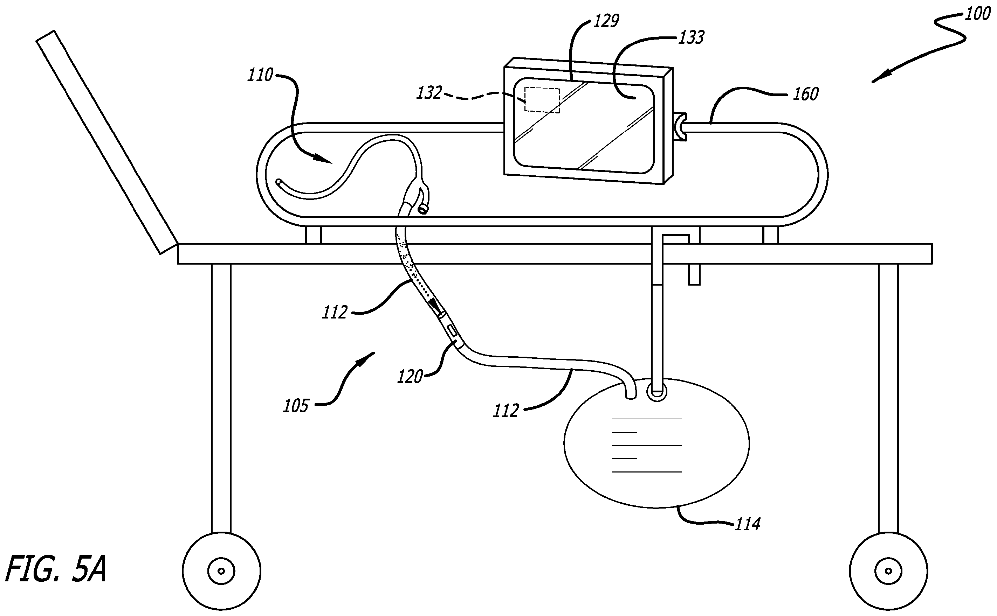

FIGS. 5A-5D illustrate the system 100 in various stages of an exemplary method 200 of monitoring the urinary output of a patient using the urinary output monitoring system 100, in accordance with some embodiments. In some embodiments, monitoring the urinary output includes measuring the flow rate of the volume of fluid excreted by the patient. It can be assumed in this method 200 that the patient is already catheterized with the urinary catheter 110 and the monitor 129 has been coupled to the securing surface 160.

As illustrated in FIG. 5A, the drainage tube 112 provides fluid communication between the urinary catheter 110 with the fluid collection bag 114. The flow meter 120 may be in line with the fluid collection bag 114 and in wireless communication with the monitor console 132, the monitor console 132 being in wireless communication with the monitor 129. Once the patient has been catheterized with the urinary catheter 110 and fluid communication has been established between the urinary catheter 110 and the fluid collection bag 114, the patient may void or excrete a volume of fluid that travels through the drainage tube 112 to the fluid collection bag 114. The flow meter 120 includes the flow meter identifier that may be communicated to the monitor console 132. The monitor console 132 may be configured to associate the received urine flow rate data (e.g., flow rate values) along with the flow meter identifier.

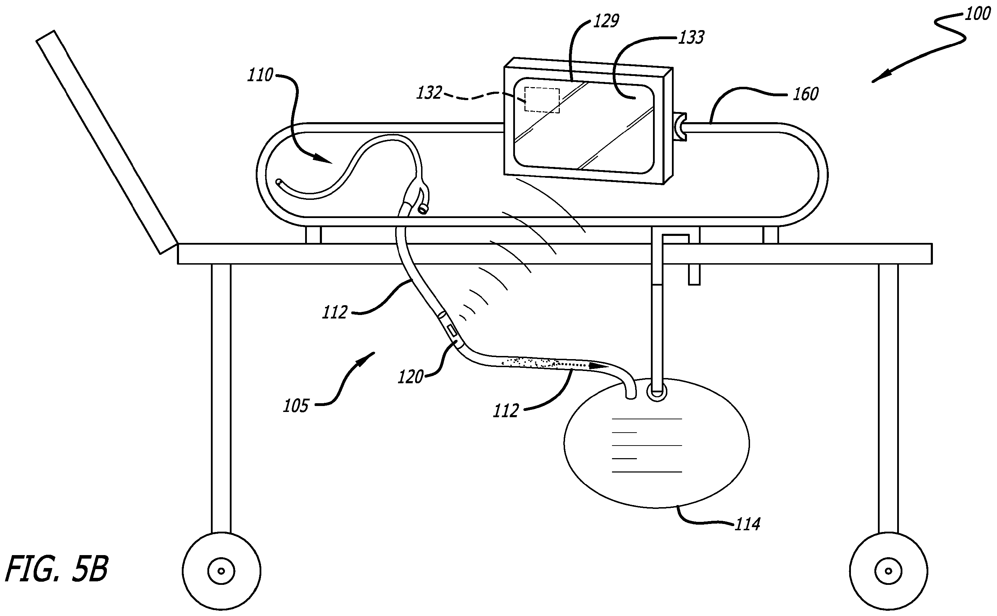

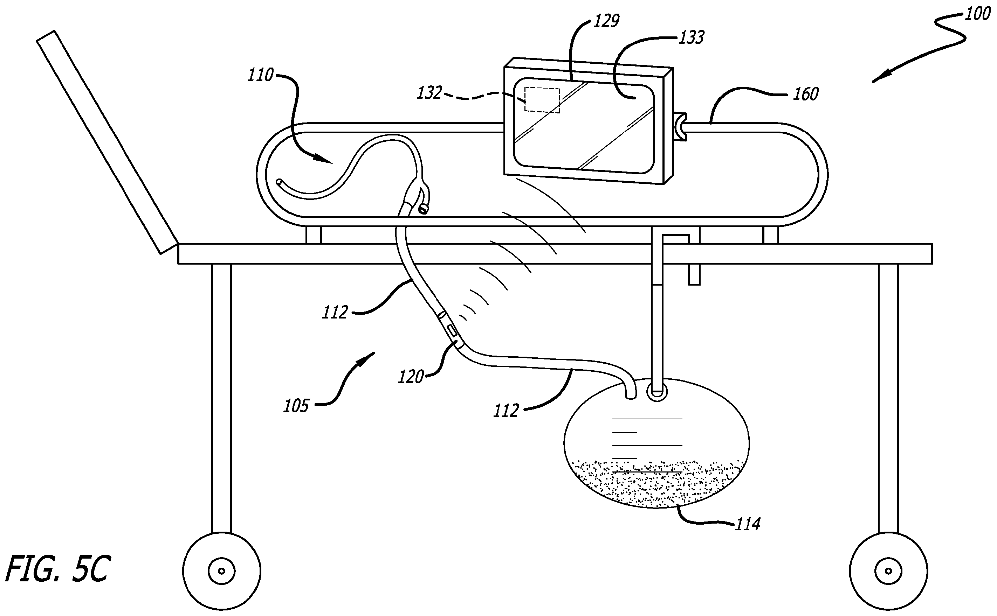

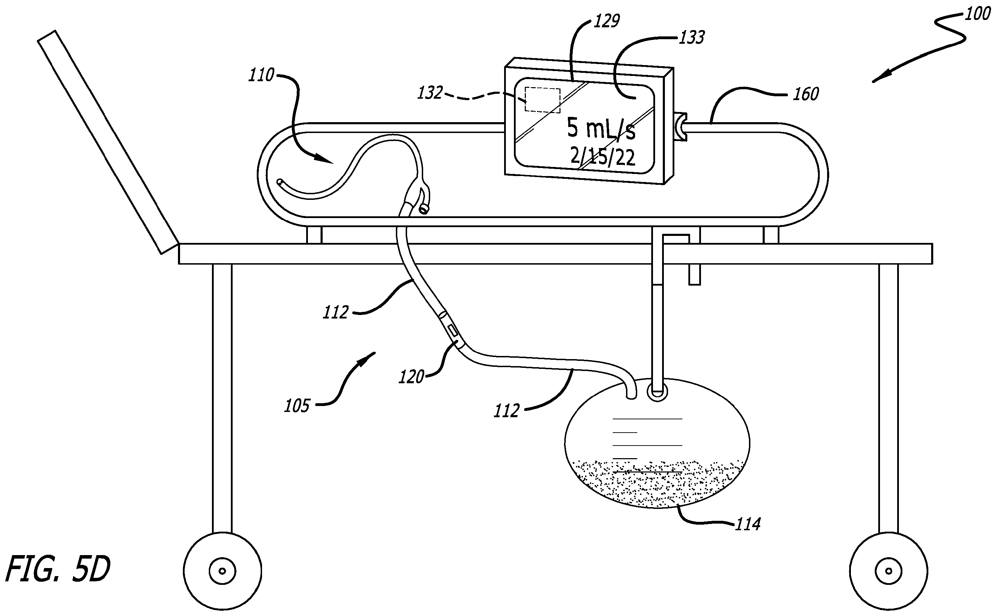

As illustrated in FIG. 5B, as the volume of fluid passes through the flow meter 120 in line with the fluid collection bag 114, the flow meter 120 may detect the volume of fluid and generate one or more flow rate values corresponding to the flow rate of the volume of fluid as the volume of fluid passes through the flow meter 120. In some embodiments, the flow meter 120 may be configured to be continually detecting for the presence of the volume of fluid or may be configured to be activated to determine the presence of the volume of fluid before the patient voids the volume of fluid. As illustrated in FIG. 5C, the flow meter 120 may wirelessly communicate the one or more flow rate values to the monitor console 132. In some embodiments, the monitor console 132 may be coupled to or integrated into the monitor 129. The monitor console 132 may be configured to receive the one or more flow rate values and correlate the one or more flow rate values with a time of day value. As illustrated in FIG. 5D, the monitor console 132 may be configured to transmit the flow rate values and time of day value to the monitor 129 to be projected onto the monitor 129. Advantageously, having a pre-connected closed automated urinary output monitoring system 100 including the flow meter 120 having the flow meter identifier ensures that the determined urine flow rate data is associated with only one patient. Furthermore, if one component of the system 100 needs to be replaced, the entire pre-connected closed system 100 is replaced and the new flow meter 120 placed in communication with the monitor console 132 so that the new urine flow rate data may be aggregated with existing patient data to continue to monitor urine output of the patient. Also having the automated urinary output monitoring system 100 including the flow meter 120 transmitting the determined urine flow rate data to the monitor console 132 allows the system 100 to travel with the patient in a hospital setting, capturing the patient's entire urinary output.

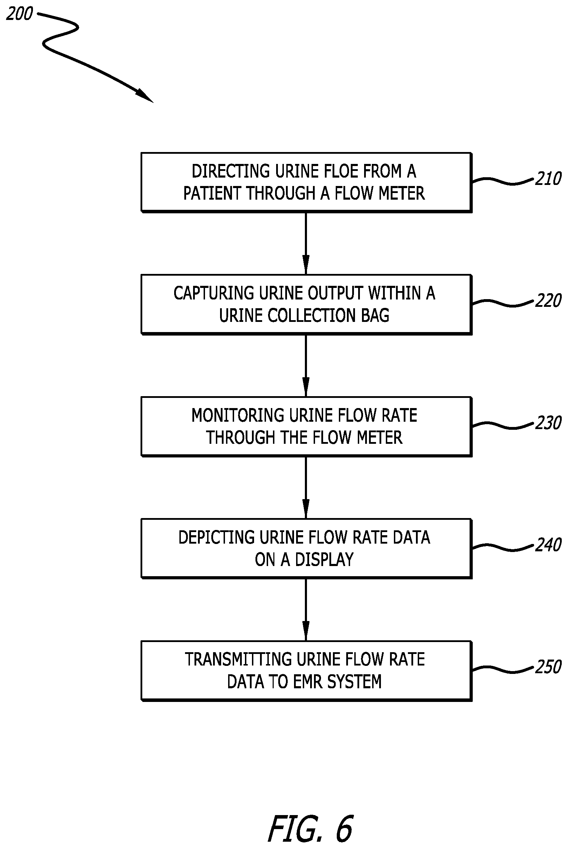

FIG. 6 illustrates a flow chart of the exemplary method 200 of monitoring urinary output of a patient as performed by the system 100, in accordance with some embodiments. The method 200 may include all or a subset of the following steps, processes, or actions. Prior to performing he method 200, a clinician may provide the system 100 and catheterize the patient. The method 200 includes directing a flow urine from a patient through the flow meter (block 210), i.e., the drainage tube directs the flow of urine through the flow meter. The method 200 further includes collecting the urine output from the patient within the urine collection bag (block 220).

The method further includes monitoring the flow rate of urine from the patient (block 230) which may include several sub-steps as further described below. Monitoring the urine flow generally include obtaining urine flow rate data from the flow meter. An additional sub-step may include wirelessly transmitting the urine flow rate data from the flow meter to a monitor of the system. Another additional sub-step may include correlating the urine flow rate data with a time of day to define correlated urine flow rate data. Another additional sub-step may include integrating the urine flow rate data with respect to time to calculate a urine output volume. Another additional sub-step may include correlating the urine flow rate data with a flow meter identifier of the flow meter.

The method 200 may further include depicting the urine flow rate data and/or other information on the display of the monitor (block 240). Depicting the urine flow rate data may include depicting the urine output volume on the display. Depicting other information may include depicting a battery level of a flow meter battery on the display. Depicting other information may include depicting the patient identification on the display.

The method 200 may further include wirelessly communicating with an EMR system (block 250). Communicating with the EMR may include transmitting the correlated urine flow rate data and the urine output volume to the EMR system. Communicating with the EMR system may also include transmitting the battery level of the flow meter battery to the EMR system. Communicating with the EMR may further include correlating the flow meter identifier with a patient identification from the EMR system.

While some particular embodiments have been disclosed herein, and while the particular embodiments have been disclosed in some detail, it is not the intention for the particular embodiments to limit the scope of the claims. Additional adaptations and/or modifications can appear to those of ordinary skill in the art, and, in broader aspects, these adaptations and/or modifications are encompassed as well. Accordingly, departures may be made from the particular embodiments disclosed herein without departing from the scope of the claims.

## Claims

1. A urinary output collection device (105), comprising:
a urinary catheter (110) coupled with a urine collection bag (114) via a drainage tube (112) extending between the urinary catheter and the urine collection bag;
a flow meter (120) in line with the drainage tube configured to determine a flow rate of urine flowing from the urinary catheter to the urine collection bag, the flow meter including a flow meter console (122) including one or more processors and a non-transitory computer-readable medium having stored thereon flow meter logic that, when executed by the one or more processors, causes flow meter operations that include:
determining urine flow rate data; and
wirelessly transmitting (i) the urine flow rate data a monitor (129); and **characterised in that** the non-transitory computer-readable medium further stores a flow meter identifier, and the flow meter logic is configured to cause wireless transmission of the flow meter identifier to the monitor (129), together with the urine flow rate data.

2. The device according to claim 1, further including a sample port (116) coupled the drainage tube, the sample port configured to enable a clinician to draw a sample of the urine from the drainage tube via a volumetric device.

3. The device according to any claim of claims 1-2, wherein the flow meter identifier includes characters selected from the group consisting of alphanumeric characters, special characters, spaces, and combinations thereof.

4. The device according to any claim of claims 1-3, wherein the flow meter console includes a battery (136).

5. The device according to any claim of claims 1-4, wherein urinary catheter, the drainage tube, and the urine collection bag define a pre-connected closed fluid system.

6. The device according to any claim of claims 1-5, wherein the flow meter is disposable.

## Patentansprüche

1. Urinausgabesammelvorrichtung (105), umfassend:
einen Urinkatheter (110), der mit einem Urinsammelbeutel (114) über einen Drainageschlauch (112) gekoppelt ist, der sich zwischen dem Urinkatheter und dem Urinsammelbeutel erstreckt;
einen Durchflussmesser (120), der sich in einer Linie mit dem Drainageschlauch befindet und konfiguriert ist, um eine Durchflussrate von Urin zu bestimmen, der von dem Urinkatheter zu dem Urinsammelbeutel fließt, wobei der Durchflussmesser eine Durchflussmesserkonsole (122) umfasst, die einen oder mehrere Prozessoren und ein nicht-transitorisches computerlesbares Medium aufweist, das eine darauf gespeicherte Durchflussmesserlogik aufweist, die, wenn sie von dem einen oder den mehreren Prozessoren ausgeführt wird, Durchflussmesservorgänge veranlasst, die Folgendes einschließen:
Bestimmen von Urindurchflussratendaten; und
drahtloses Übertragen (i) der Urindurchflussratendaten an einen Monitor (129); und **dadurch gekennzeichnet, dass** das nicht-transitorische computerlesbare Medium weiter eine Durchflussmesserkennung speichert, und die Durchflussmesserlogik konfiguriert ist, um eine drahtlose Übertragung der Durchflussmesserkennung an den Monitor (129) zusammen mit den Urindurchflussratendaten zu veranlassen.

2. Vorrichtung nach Anspruch 1, weiter einschließend einen mit dem Drainageschlauch gekoppelten Probenanschluss (116), wobei der Probenanschluss konfiguriert ist, um es einem Arzt zu ermöglichen, eine Urinprobe aus dem Drainageschlauch über eine volumetrische Vorrichtung zu entnehmen.

3. Vorrichtung nach einem der Ansprüche 1-2, wobei die Durchflussmesserkennung Zeichen einschließt, die aus der Gruppe bestehend aus alphanumerischen Zeichen, Sonderzeichen, Leerzeichen und Kombinationen davon ausgewählt sind.

4. Vorrichtung nach einem der Ansprüche 1-3, wobei die Durchflussmesserkonsole eine Batterie (136) einschließt.

5. Vorrichtung nach einem der Ansprüche 1-4, wobei der Urinkatheter, der Drainageschlauch und der Urinsammelbeutel ein vorverbundenes geschlossenes Fluidsystem definieren.

6. Vorrichtung nach einem der Ansprüche 1-5, wobei der Durchflussmesser entsorgbar ist.

## Revendications

1. Dispositif de collecte de sortie urinaire (105), comprenant :
un cathéter urinaire (110) couplé à une poche de collecte d'urine (114) via un tube de drainage (112) s'étendant entre le cathéter urinaire et la poche de collecte d'urine ;
un débitmètre (120) en ligne avec le tube de drainage configuré pour déterminer un débit d'urine s'écoulant du cathéter urinaire vers la poche de collecte d'urine, le débitmètre comprenant une console de débitmètre (122) incluant un ou plusieurs processeurs et un support lisible par ordinateur non transitoire sur lequel est stockée une logique de débitmètre qui, lorsqu'elle est exécutée par le ou les processeurs, provoque des opérations de débitmètre qui comprennent :
la détermination des données de débit d'urine ; et
la transmission sans fil (i) des données de débit d'urine à un moniteur (129) ; et **caractérisé en ce que** le support informatique lisible par ordinateur non transitoire stocke en outre un identifiant de débitmètre, et la logique du débitmètre est configurée pour provoquer la transmission sans fil de l'identifiant du débitmètre au moniteur (129), avec les données de débit d'urine.

2. Dispositif selon la revendication 1, incluant en outre un orifice d'échantillonnage (116) couplé au tube de drainage, l'orifice d'échantillonnage étant configuré pour permettre à un clinicien de prélever un échantillon d'urine à partir du tube de drainage via un dispositif volumétrique.

3. Dispositif selon l'une quelconque des revendications 1 à 2, dans lequel l'identifiant du débitmètre comprend des caractères sélectionnés parmi le groupe constitué de caractères alphanumériques, de caractères spéciaux, d'espaces et de combinaisons de ceux-ci.

4. Dispositif selon l'une quelconque des revendications 1 à 3, dans lequel la console du débitmètre inclut une batterie (136).

5. Dispositif selon l'une quelconque des revendications 1 à 4, dans lequel le cathéter urinaire, le tube de drainage et la poche de collecte d'urine définissent un système de fluide fermé pré-connecté.

6. Dispositif selon l'une quelconque des revendications 1 à 5, dans lequel le débitmètre est jetable.
